# EUROPEAN PATENT APPLICATION

(11) **EP 4 613 747 A1**
(43) Date of publication of application: **10.09.2025**
(21) Application number: 23885105.9
(22) Date of filing: 03.11.2023
(51) Int. Cl.: C07D 471/04

(54) **METHOD FOR PREPARING FINERENONE BY MEANS OF RESOLVING RACEMATE WITH DIASTEREOMERIC TARTARIC ESTER**

(30) Priority: 04.11.2022 CN 202211374807
(71) Applicant: Nanjing Vcare Pharmatech Co., Ltd., Nanjing, Jiangsu 210061 (CN)
(72) Inventor: LIU, Song, Nanjing, Jiangsu 210061 (CN); LEI, Xiantao, Nanjing, Jiangsu 210061 (CN); REN, Yingmei, Nanjing, Jiangsu 210061 (CN); LIU, Xuefang, Nanjing, Jiangsu 210061 (CN); GONG, Yanchun, Nanjing, Jiangsu 210061 (CN)
(74) Representative: Ullrich & Naumann PartG mbB
(86) International application number: PCT/CN2023/129647
(87) International publication number: WO 2024/094181

(57) **Abstract**

The present invention provides a method for preparing finerenone by means of resolving a racemate with a diastereomeric tartaric ester, and also relates to diastereomer salts (VIIa), (VIIb), (VIIc) and/or (VIId). The resolving method uses a chiral substituted tartaric ester represented by general formula (VIa) or (VIb) as a resolving agent to resolve a racemate represented by formula (V) to obtain a compound represented by formula (Va) and/or (Vb). The racemate is firstly added into a solvent mixture to be heated until completely dissolved; then the resolving agent is added to obtain a homogeneous system; and resolution is performed by stirring for crystallization. The optimization of the feeding sequence can ensure sufficient salt formation, thereby greatly shortening the time for crystallization. In a basic dissociation step, a crude product with very high chemical purity and enantiomeric purity can be obtained by means of one-time dissociation, and the content of the tartaric ester as the resolving agent is less than 0.05%, such that the three wastes caused by multiple dissociations are avoided, and the production cost is greatly reduced.

## Description

The present invention claims the priority of the prior application filed with the China National Intellectual Property Administration (CNIPA) on November 4, 2022, with Patent Application No. 202211374807.2 and entitled "Method for preparing phenerenone by means of resolving racemate with diastereomeric tartaric ester". The entirety of the above prior application is incorporated by reference into the present invention.

### Field of the Invention

The present invention belongs to the field of pharmaceutical technology, and in particular relates to a method for preparing finerenone by means of resolving a racemate with a diastereomeric tartaric ester, and also relates to diastereomer salts (VIIa), (VIIb), (VIIc) and/or (VIId).

### Background of the Invention

The term "finerenone" refers to the compound (4S)-4-(4-cyano-2-methoxyphenyl)-5-ethoxy-2,8-dimethyl-1,4-dihydro-1,6-naphthyridine-3-carboxamide, i.e., a compound represented by formula (Va), and the compound represented by formula (V) is the racemate of finerenone,

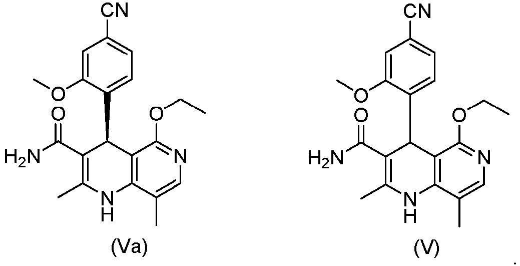

The expression "enantiomer of finerenone" or "enantiomer of a compound represented by formula (V)" refers to compounds represented by formula (Va) and (Vb),

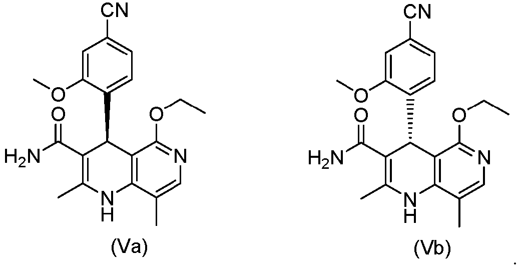

Finerenone (Va) acts as a non-steroidal antagonist of mineralocorticoid receptors and can be used as an agent for preventing and/or treating cardiovascular and renal diseases such as heart failure and diabetic nephropathy. The compounds of formula (V) or (Va) and their preparation methods are recited in WO2008/104306 and WO2016/016287A1, and Bärfacker L, Kuhl A, Hillisch A, et al. Discovery of BAY 94 - 8862: a nonsteroidal antagonist of the mineralocorticoid receptor for the treatment of cardiorenal diseases[J]. ChemMedChem, 2012, 7(8): 1385-1403. In order to obtain an optically pure compound of formula (V), the racemic mixture of amide (V) must be separated into enantiomers (Va) and (Vb); 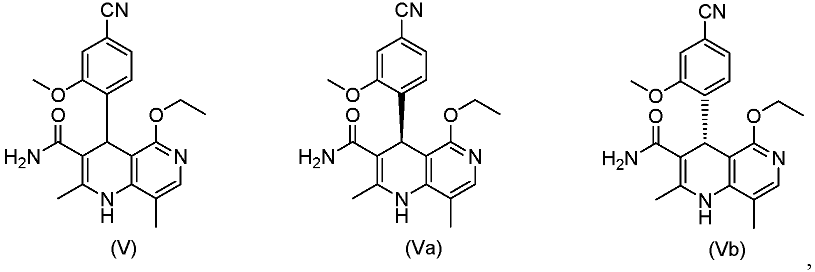 since only formula (Va) is active

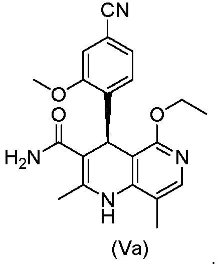

Chinese patent application CN112041318A recites the resolution of the racemate represented by formula (V), which is resolved into (Va) and/or (Vb) using a chiral substituted tartaric ester of general formula (IIIa) or (IIIb). However, the patent only recites the use of D-dibenzoyltartaric acid as a resolving agent, and the actual target isomer recovery rate is about 90%, with a large resolution loss. Furthermore, in the step of treating the diastereoisomer salt with a base, multiple dissociations are usually required to reduce the content of the chiral substituted tartaric ester as the resolving agent to less than 0.15%.

### SUMMARY OF THE INVENTION

The present invention provides a method for resolving a racemate represented by formula (V), which uses a chiral substituted tartaric ester represented by general formula (VIa) or (VIb) as a resolving agent to resolve a racemate represented by formula (V) to obtain a compound represented by formula (Va) and/or (Vb), 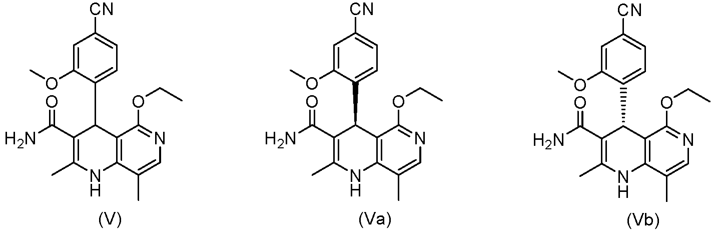 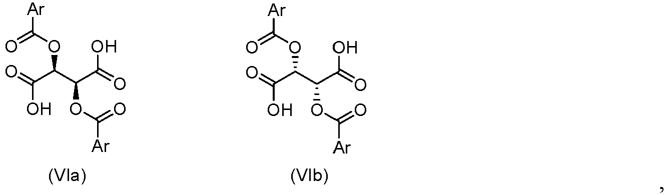 wherein Ar is selected from: 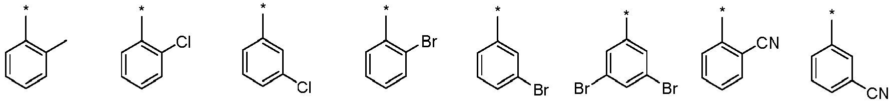 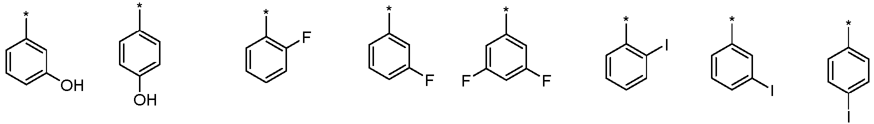 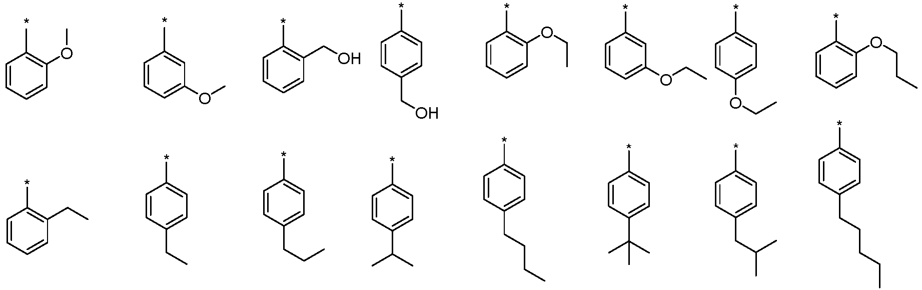 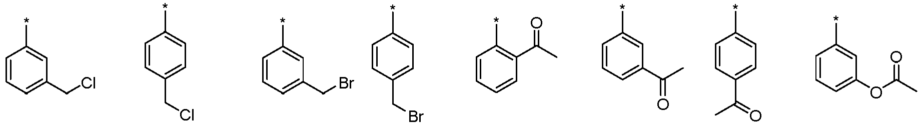 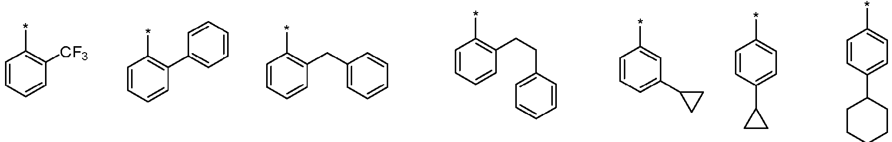 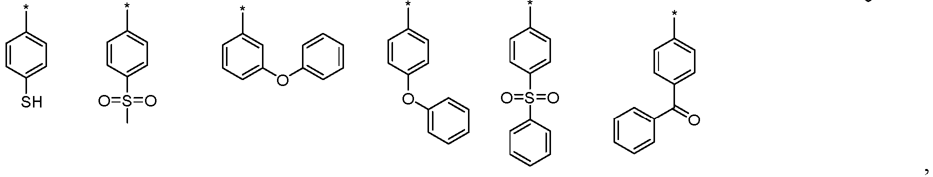 wherein * represents an attachment point.

Another aspect of the present invention provides a method for preparing (4S)-4-(4-cyano-2-methoxyphenyl)-5-ethoxy-2,8-dimethyl-1,4-dihydro-1,6-naphthyridine-3-carboxamide represented by formula (Va), which uses a chiral substituted tartaric ester represented by formula (VIa) as a resolving agent to resolve a racemate represented by formula (V), 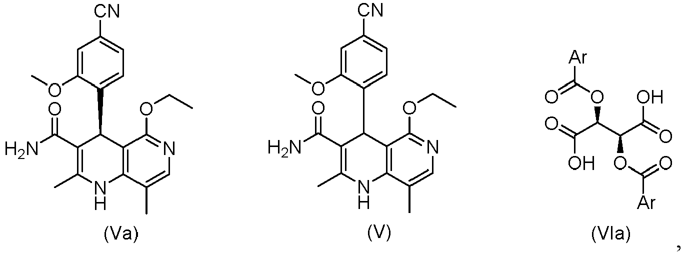 wherein Ar is selected from: 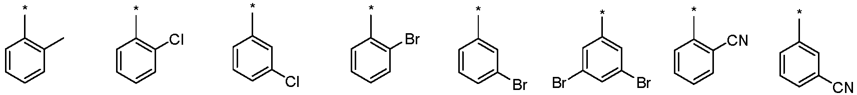 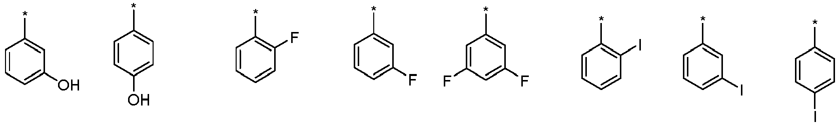 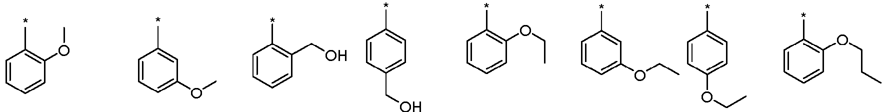 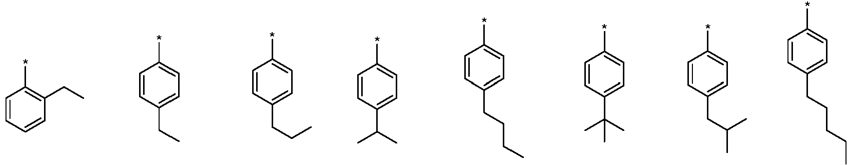 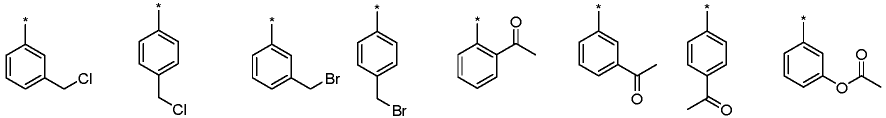 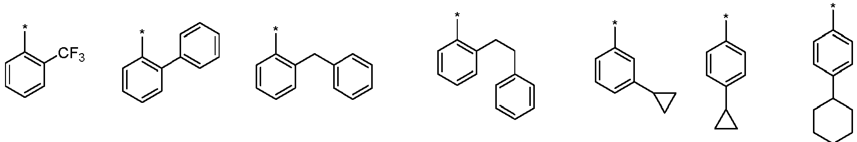 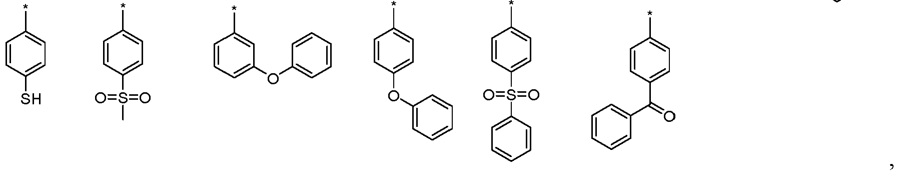 wherein * represents an attachment point.

Preferably, Ar is selected from: 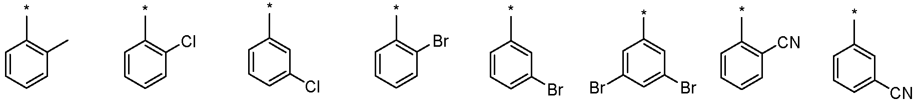 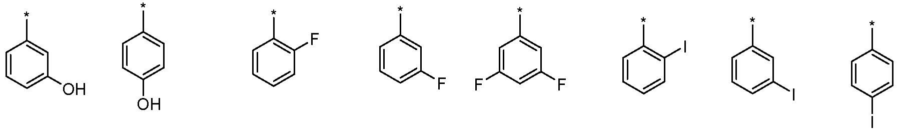 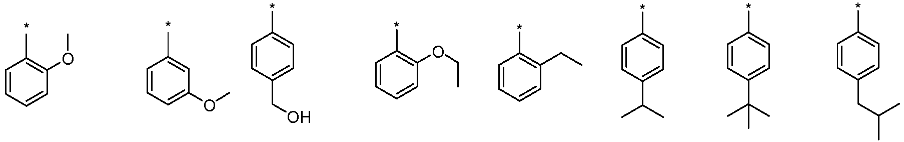 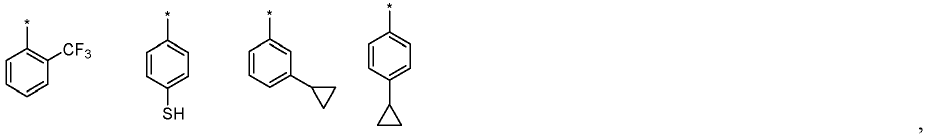 wherein * represents an attachment point.

In the embodiments of the present invention, the resolving agent is selected from:

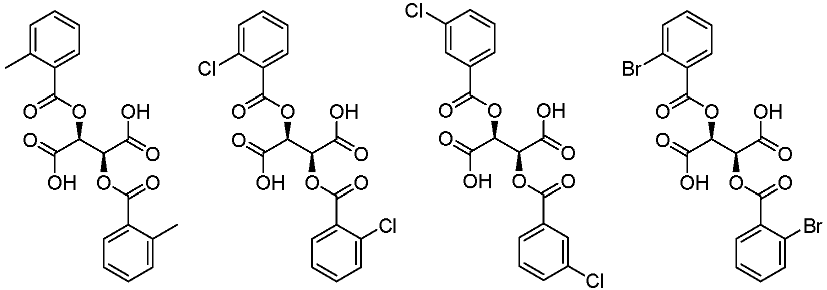

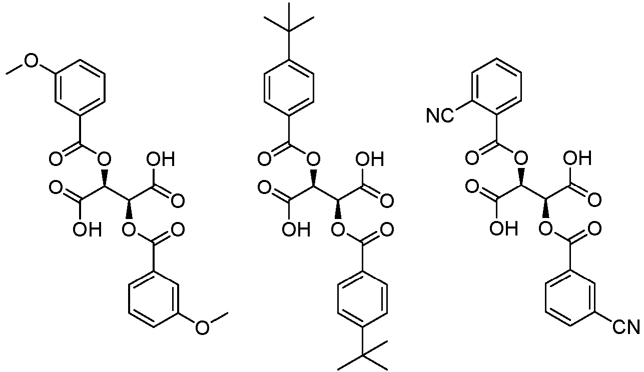

Preferably, the resolving agent is selected from:

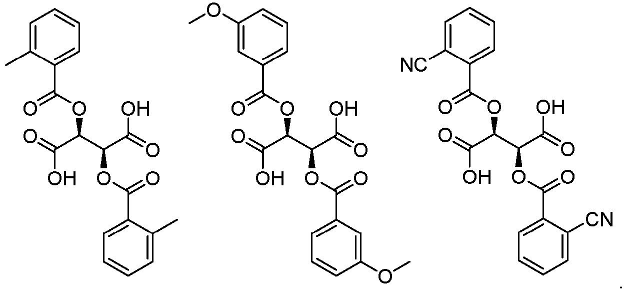

Particularly preferably, the resolving agent is:

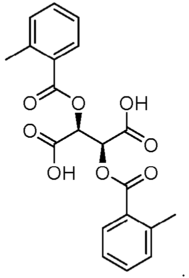

Another aspect of the present invention also provides diastereomer salts represented by the following formulae: 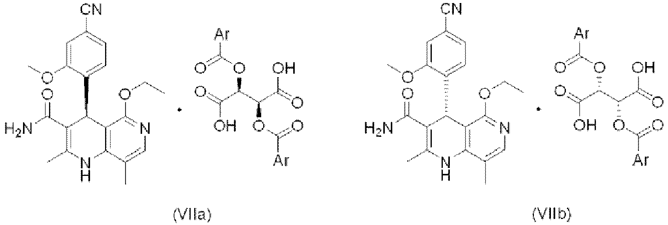 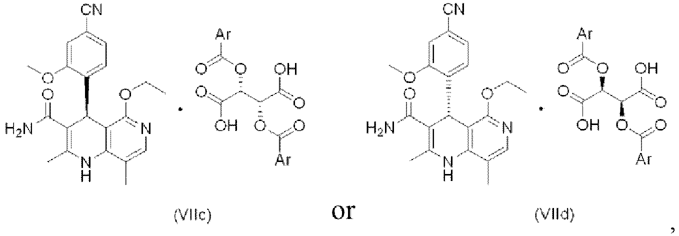 wherein Ar is selected from: 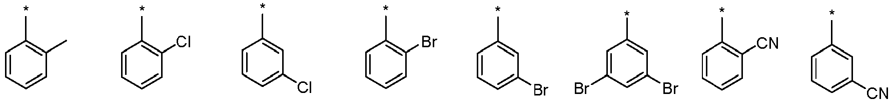 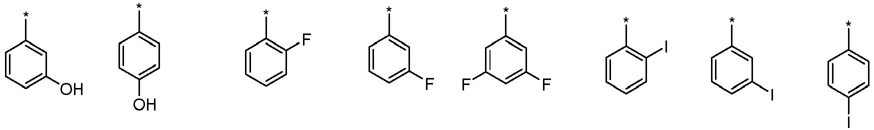 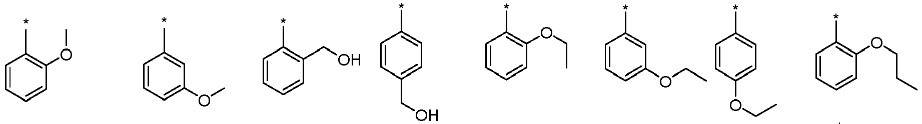 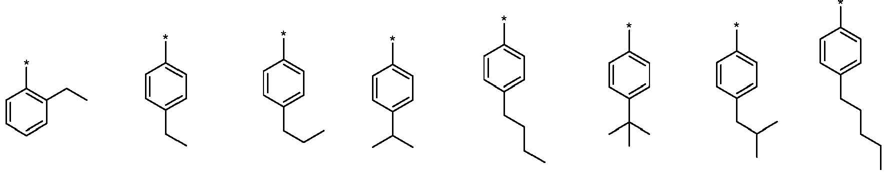 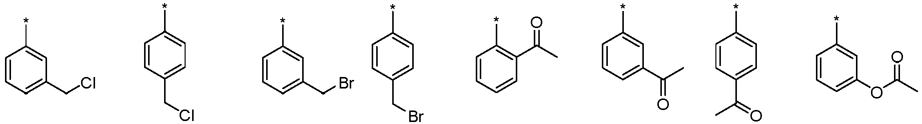 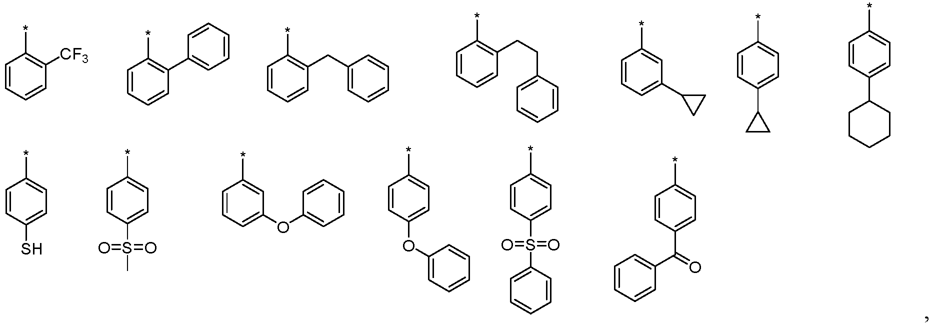 wherein * represents an attachment point.

The preparation of the diastereomer salts (VIIa to VIId) is carried out as follows:

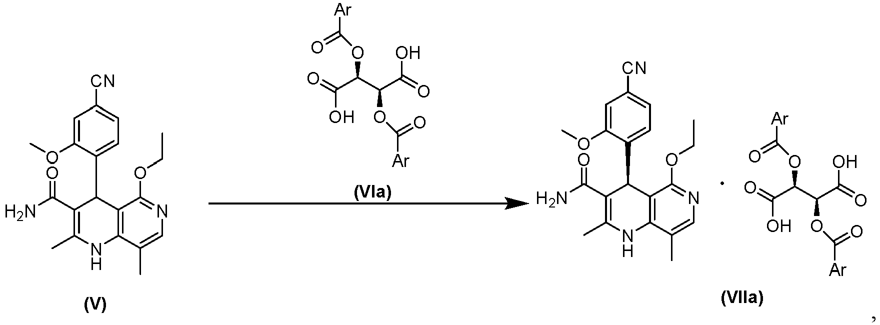

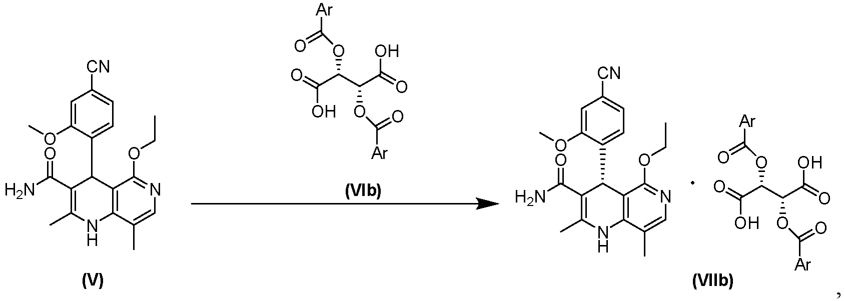

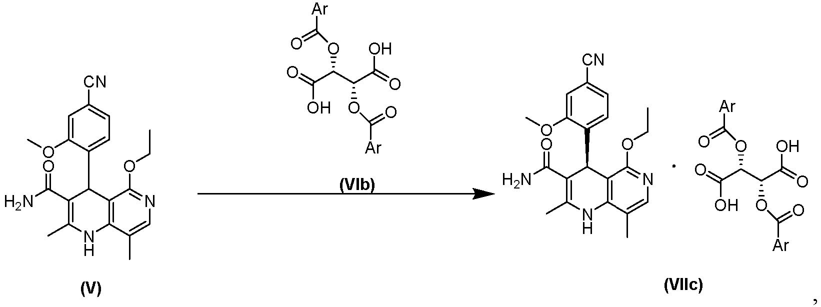

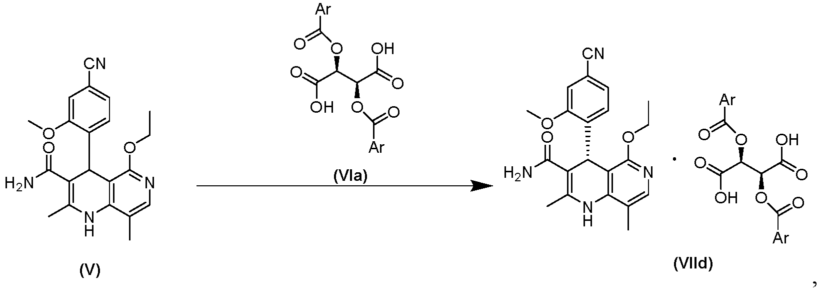

Reaction of a racemate represented by formula (V) with a chiral substituted tartaric ester represented by formula (VIa) or (VIb) results in the formation of four options of diastereomer salts (VIIa, VIIb, VIIc and VIId). If the racemate represented by formula (V) is reacted with the chiral substituted tartaric ester represented by formula (VIa), the diastereomer salt of formula (VIIa) is obtained, wherein the enantiomer in the S configuration preferentially participates in the salt formation. The diastereomer salt of formula (VIIa) precipitates almost quantitatively from the solution and can then be separated from the solution, for example by filtration, where the enantiomer in the R configuration remains in the solution. The mirror-image salt of formula (VIIb) is prepared by reacting the racemate represented by formula (V) with a chiral substituted tartaric ester represented by formula (VIb), wherein the enantiomer in the R configuration preferentially participates in the salt formation. The precipitated diastereomer salt can be separated almost quantitatively, where the S-enantiomer remains in the solution.

The stoichiometric ratio of (V) to (VIa)/(VIb) and the choice of solvents can be used to optimize the yield and enantiomeric purity.

Finerenone (Va) has the S configuration. The tartaric ester in the S,S-configuration is preferably used for racemate resolution, and a diastereomer salt of the S-enantiomer is preferably formed.

0.5 to 3.0 equivalents of the chiral substituted tartaric ester represented by formula (VIa) or (VIb) is used as a resolving agent for optical resolution, and it is preferably 0.5 to 1.5 equivalents, more preferably 0.5 to 1.0 equivalents, and most preferably 0.55 equivalents.

The diastereomer salt is formed in an organic solvent or a solvent mixture consisting of water and a water-miscible organic solvent.

Examples of a suitable organic solvent include ethanol, methanol, isopropanol, n-propanol, dichloromethane, tetrahydrofuran, acetonitrile, methyl tert-butyl ether or acetone, but ethanol is preferably used. In addition, the following solvents are used: methanol/water 3:1; methanol/acetonitrile 1:1; isopropanol/water 3:1; tetrahydrofuran/water 3:1; and acetonitrile/water 3:1. Herein, the ratio in the solvent ratios refers to the ratio of volume to volume (v/v). For example, a solvent mixture consisting of ethanol/water 3:1 comprises 60 mL of ethanol and 20 mL of water. Therefore, the volumes are based on the total volume of the solvent.

The optical resolution is preferably carried out in ethanol/water, wherein the mixing ratio (v/v) is in the range of ethanol:water = 1:1 to 9:1. However, it is preferred to use a mixture of ethanol:water = 3:1 to 9:1. A mixture of ethanol:water = 3:1 is particularly preferred. The mixture can be prepared in advance, or prepared in situ after all components are placed in a container. The solvent mixture may be used in 5- to 30-fold excess relative to the racemate represented by formula (V), i.e. 5 to 30 L of the solvent mixture per 1 Kg of the racemate. A 5- to 15-fold excess is preferred.

Optical resolution is usually carried out as follows: first, the racemate represented by formula (V) is added to a solvent mixture at room temperature, then heated to 0 to 78°C, but preferably 70 to 78°C, and stirring is continued at 70 to 78°C until completely dissolved, it being most preferably at a temperature of 75°C. Subsequently, the resolving agent is added to the above system and stirred at 70 to 78°C for 0.5 to 3 hours, preferably 0.5 to 1 hour. Then, the mixture is cooled to 0 to 30°C, preferably 15 to 25°C, over 3 to 15 hours, preferably 3 to 5 hours. Thereafter, stirring is continued at 15 to 25°C for 12 to 24 hours, preferably 12 to 18 hours, very preferably 12 to 14 hours. In the present invention, the racemate is firstly added into a solvent mixture to be heated until completely dissolved; then the resolving agent is added to obtain a homogeneous system; and resolution is performed by stirring for crystallization. The optimization of the feeding sequence can ensure sufficient salt formation, thereby greatly shortening the time for crystallization.

The precipitated diastereomer salts (VIIa), (VIIb), (VIIc) and/or (VIId) are subsequently separated.

The separation is carried out by methods known to those skilled in the art, for example by filtration or using a centrifuge. A filter cake obtained in this way can be washed once or several times with a solvent or solvent mixture. Then,drying is carried out under reduced pressure at a temperature (45 to 65°C, preferably 55°C).

The diastereomer salts can be prepared in very high chemical purity using the above steps. The enantiomeric excess of the diastereomer salts is generally > 97.0% e.e.

The diastereomer salts are not necessarily dried, and they can be used wet in the next process.

In the next step, the diastereomer salts are treated with a base. To prepare the chiral compounds (Va) and (Vb), the diastereomer salt of formula (VIIa), (VIIb), (VIIc) or (VIId) must be treated with a base, and the corresponding tartaric ester of formula (VIa) or (VIb) remains in the solution in the form of a salt.

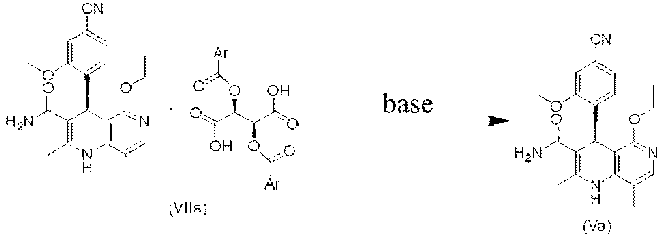

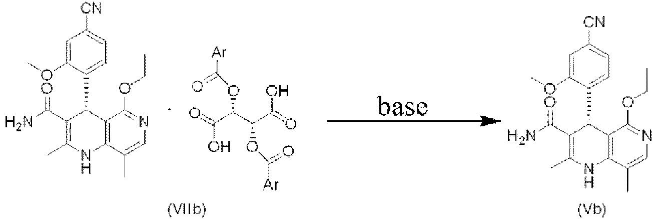

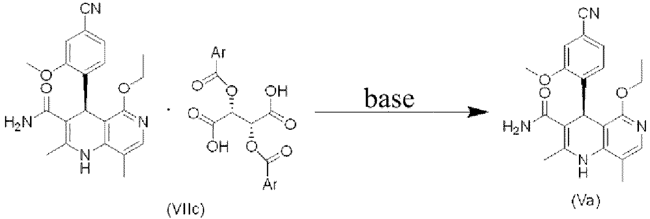

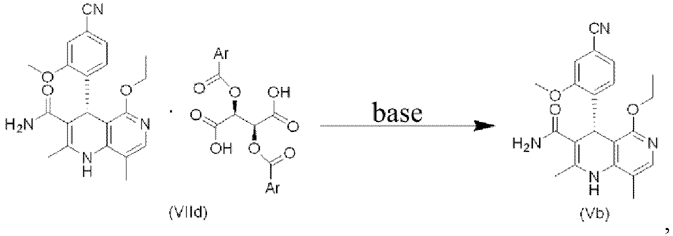

In the context of the present invention, a suitable base is an inorganic base or an organic base. For the inorganic base, ammonia, sodium hydroxide, lithium hydroxide, potassium hydroxide, ammonium carbonate, sodium carbonate, potassium carbonate, lithium carbonate, ammonium hydrogen carbonate, sodium hydrogen carbonate, potassium hydrogen carbonate, ammonium phosphate, sodium phosphate or potassium phosphate can be used. Sodium hydroxide, sodium phosphate or potassium phosphate is preferably used. Sodium phosphate or potassium phosphate is particularly preferably used. It is important that the inorganic base can be used in anhydrous form or in the form of a hydrate thereof; for example, sodium phosphate anhydrate and sodium phosphate hydrate can be used successfully. For the organic base, an aliphatic or aromatic base such as methylamine, dimethylamine, trimethylamine, ethylamine, diethylamine, triethylamine, propylamine, isopropylamine, 1,3-propylenediamine, 1,2-propylenediamine, tripropylamine, cyclohexylamine, dicyclohexylamine, N,N-diisopropylethylamine, aniline, diphenylamine, triphenylamine, monoethanolamine, diethanolamine, triethanolamine, N-methylmorpholine, pyridine, sodium tert-butoxide, potassium tert-butoxide, sodium ethoxide or sodium methoxide can be used. Triethylamine, N-methylmorpholine, N,N-diisopropylethylamine or aniline are preferred.

In an embodiment of the present invention, an inorganic base is added at a temperature of 0 to 60°C to adjust the pH of the system to 6.9 to 9.5.

In an embodiment of the present invention, the organic base is added at a temperature of 0 to 60°C, and the organic base equivalent is 1.5 to 6.0.

The target compound (Va) or (Vb) is released in a mixture of water and a water-miscible organic solvent such as ethanol, isopropanol, methanol or acetone, preferably ethanol. It has been found that it is advantageous to use a mixture of water and ethanol in a mixing ratio (v/v) of ethanol:water = 1:20 to 1:3. However, it is preferred to use a mixture of ethanol:water = 1:19. The mixture can be prepared in advance, or prepared in situ after all components are placed in a container. The amount of the mixture used may be 8 to 35 times the amount of the diastereomer salt (VIIa or VIIb or VIIc or VIId) used, i.e. for example, 8 to 35 L of the mixture in 1 Kg. It is preferred to use 15 to 35 times the amount of the mixture, more preferably 25 to 35 times the amount of the mixture, and most preferably 30 times the amount of the mixture.

The target compound (Va) or (Vb) is released by first adding a diastereomer salt (VIIa or VIIb or VIIc or VIId) to a mixed solvent at 0°C to 80°C, preferably 20°C to 55°C, and then adding an organic or inorganic base (in solid form or in solution, preferably in water) to adjust the pH to 6.9 to 9.5, preferably 7.1 to 8.0, and particularly preferably 7.5. The base may be added very quickly (over a few minutes), or very slowly (over a few hours), for example, over 3 minutes up to 2 hours. In any case a more rapid addition is preferred. Metered addition over 3 to 30 minutes is preferred. This can be achieved by means of a pH meter installed in the reactor, which is used for controlled regulation and gradually metered addition of the base. It has been found that after establishing the pH, it is advantageous to continue stirring again at 10°C to 80°C, preferably 20°C to 65°C, preferably 45°C to 55°C. The duration of continued stirring may be 1 to 20 hours, preferably 2 to 6 hours, and more preferably 2 to 3 hours. The mixture is then cooled to 20°C to 30°C and may then be stirred again for 1 to 40 hours, preferably 3 to 24 hours, and more preferably 10 to 18 hours.

The separation is carried out by methods known to those skilled in the art, for example by filtration or using a centrifuge. A filter cake obtained in this way can be washed once or more than once with a solvent or solvent mixture. Then, drying is carried out under reduced pressure (preferably < 100 mbar) at a temperature (50 to 80°C, preferably 55°C).

A crude product of very high chemical purity can be prepared using the above steps. The enantiomeric excess of the crude products (Va) and (Vb) is usually > 97% e.e., preferably > 99% e.e.; and the content of the tartaric ester as the resolving agent is < 0.05%.

As a particularly preferred method, in particular for implementation on an industrial scale, D-di-o-methylbenzoyltartaric acid (VI-1) is used as the resolving agent:

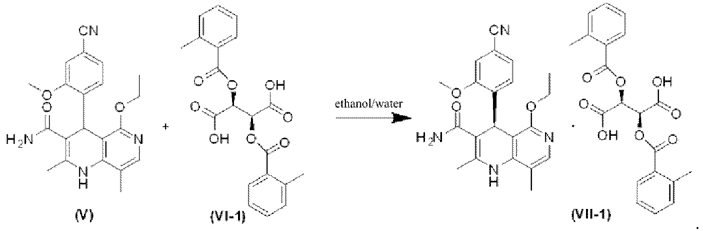

The racemate resolution is preferably carried out in an ethanol/water mixture. Subsequently, (Va) is released using sodium phosphate or sodium hydroxide as base, preferably in an ethanol/water mixture:

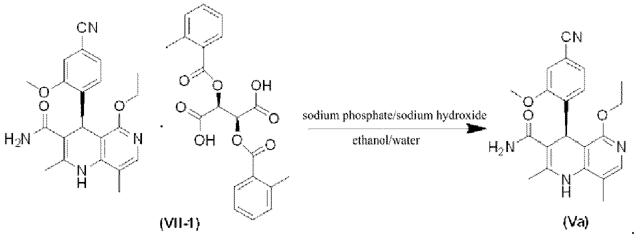

The present invention can also separate the target enantiomer from the mother liquor. Herein, a suitable diastereomer salt (VIa), (VIb), (VIc) or (VId) is prepared from formula (Va) or (Vb), and then separated by filtration, and then the pH of the mother liquor containing the corresponding other diastereomer is adjusted to pH > 7, preferably pH = 7.5 by adding a base. The base is ammonia, sodium hydroxide, lithium hydroxide, potassium hydroxide, ammonium carbonate, sodium carbonate, potassium carbonate, lithium carbonate, ammonium bicarbonate, sodium bicarbonate, potassium bicarbonate, sodium phosphate, potassium phosphate, or ammonium phosphate. Sodium hydroxide, sodium phosphate or potassium phosphate is preferably used, and sodium phosphate or potassium phosphate is particularly preferably used. The organic solvent, preferably ethanol, is then distilled off at atmospheric pressure or, more gently, at reduced pressure; and this causes the corresponding other enantiomer to precipitate. The product is filtered out, washed with water or a water/organic solvent mixture and dried.

Beneficial effects: In the present invention, the racemate is firstly added into a solvent mixture to be heated until completely dissolved; then the resolving agent is added to obtain a homogeneous system; and resolution is performed by stirring for crystallization. The optimization of the feeding sequence can ensure sufficient salt formation, thereby greatly shortening the time for crystallization. In addition, for the diastereomer salts of the present invention, in a basic dissociation step, a crude product with very high chemical purity and enantiomeric purity can be obtained by means of one-time dissociation, and the content of the tartaric ester as the resolving agent is less than 0.05%, such that the three wastes caused by multiple dissociations are avoided, and the production cost is greatly reduced.

### Brief Description of the Drawings

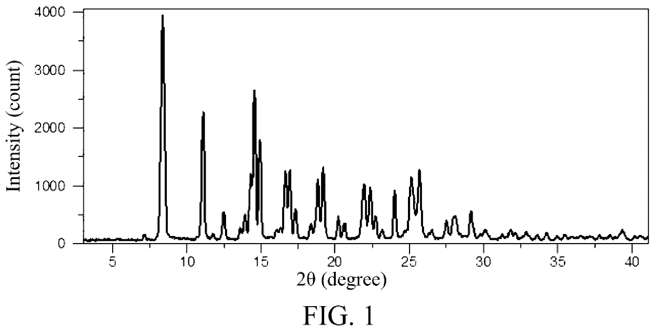

FIG. 1 is an X-ray powder diffraction (XRPD) spectrum of the compound of formula (VII-1).

### DETAILED DESCRIPTION OF THE INVENTION

Abbreviations and acronyms for reagents are as follows:
ethanol (EtOH), dimethyl sulfoxide (DMSO), (yield) of theoretical value (of th.), high performance liquid chromatography (HPLC), 1H nuclear magnetic resonance spectrum (1H-NMR), room temperature (RT), X-ray powder diffraction (XRPD), differential scanning calorimetry (DSC), thermogravimetric analysis (TGA), hour (h), and volume (vol).

### Example 1

### Example 1a

### Preparation of diastereomer salt (VII-1):

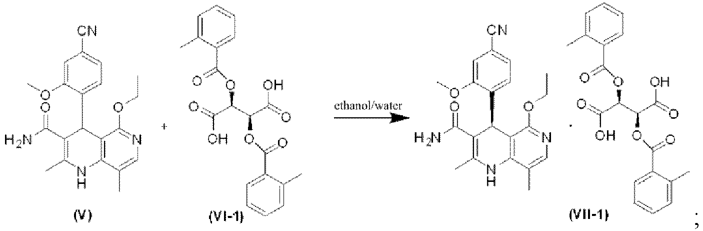

100 g (0.2642 mol) of racemic finerenone (V) was duspended in a mixture of 900 mL of ethanol and 300 mL of water, and heated to 75 °C and stirred until completely dissolved. 56.15 g (0.1453 mol) of D-di-o-methylbenzoyltartaric acid was added using a solid glass funnel and stirred at 75 °C for 1.0 h for crystallization. The mixture was then cooled to 25 °C over 3 hours and stirred at this temperature for 16 hours, and the precipitated crystals were filtered out and washed twice with 200 mL of ethanol/water (3:1, v/v). The product was dried under reduced pressure at 55 °C.

Yield: 100.2 g (99.2% of th.) as off-white crystalline powder.

### Analysis results:

enantiomeric purity (e.e.%): 97.2% e.e.; ¹H-NMR (400 MHz, DMSO-d6): δ= 7.90 - 7.85 (m, 2H), 7.70 (s, 1H), 7.58 - 7.52 (m, 3H), 7.42 - 7.35 (m, 5H), 7.29 (dd, J = 7.9, 1.5 Hz, 1H), 7.16 (d, J = 7.9 Hz, 1H), 6.73 (d, J = 33.4 Hz, 2H), 5.88 (s, 2H), 5.38 (s, 1H), 4.01 (qt, J = 6.7, 3.4 Hz, 2H), 3.83 (s, 3H), 2.55 (s, 6H), 2.19 (s, 3H), 2.13 (s, 3H), 1.05 (t, J = 7.0 Hz, 3H).

The off-white crystalline powder (VII-1) obtained according to Example 1a was characterized by X-ray powder diffraction at room temperature. The results are shown in FIG. 1 and Table 1.

**Table 1:**

| 20 (°) | d (Å) | Height (counts) | I/I₀ (%) | FWHM (°) |
|---|---|---|---|---|
| 7.128 | 12.3922 | 63 | 2.5 | 0.211 |
| 8.332 | 10.6035 | 2507 | 100.0 | 0.317 |
| 11.074 | 7.9834 | 1491 | 59.5 | 0.211 |
| 12.479 | 7.0873 | 346 | 13.8 | 0.211 |
| 13.569 | 6.5207 | 157 | 6.3 | 0.158 |
| 13.900 | 6.3660 | 297 | 11.8 | 0.158 |
| 14.567 | 6.0760 | 1735 | 69.2 | 0.158 |
| 14.920 | 5.9328 | 1161 | 46.3 | 0.211 |
| 16.608 | 5.3334 | 809 | 32.3 | 0.158 |
| 16.934 | 5.2315 | 822 | 32.8 | 0.158 |
| 17.296 | 5.1228 | 349 | 13.9 | 0.211 |
| 18.331 | 4.8358 | 183 | 7.3 | 0.211 |
| 18.810 | 4.7138 | 685 | 27.3 | 0.211 |
| 19.160 | 4.6285 | 890 | 35.5 | 0.158 |
| 20.202 | 4.3922 | 280 | 11.2 | 0.158 |
| 20.604 | 4.3073 | 205 | 8.2 | 0.211 |
| 21.927 | 4.0502 | 643 | 25.7 | 0.264 |
| 22.349 | 3.9747 | 622 | 24.8 | 0.158 |
| 22.702 | 3.9137 | 275 | 11.0 | 0.158 |
| 23.127 | 3.8428 | 101 | 4.0 | 0.211 |
| 23.972 | 3.7092 | 593 | 23.7 | 0.158 |
| 25.032 | 3.5545 | 624 | 24.9 | 0.317 |
| 25.657 | 3.4693 | 838 | 33.4 | 0.211 |
| 26.436 | 3.3689 | 82 | 3.3 | 0.317 |
| 27.476 | 3.2436 | 211 | 8.4 | 0.264 |
| 27.936 | 3.1913 | 268 | 10.7 | 0.211 |
| 29.119 | 3.0642 | 342 | 13.7 | 0.211 |
| 30.131 | 2.9635 | 104 | 4.2 | 0.422 |
| 31.817 | 2.8103 | 114 | 4.6 | 0.211 |
| 32.881 | 2.7217 | 61 | 2.4 | 0.422 |
| 33.566 | 2.6677 | 40 | 1.6 | 0.317 |
| 34.228 | 2.6176 | 66 | 2.6 | 0.211 |
| 39.324 | 2.2893 | 99 | 4.0 | 0.317 |

### Example 1b

### Preparation of crude product (Va):

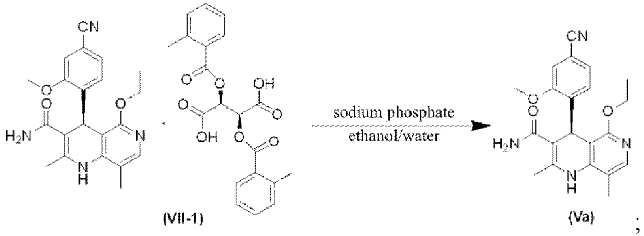

100 g of the compound (VII-1) prepared in Example 1a was suspended in a mixture consisting of 150 mL of ethanol and 2850 mL of water. Subsequently, 356.5 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 200 mL of ethanol/water (5:95, v/v). The product was dried under reduced pressure at 55 °C. Yield: 47.0 g (95.0% of th.) as off-white crystalline powder.

Analysis results:
enantiomeric purity (e.e.%): 97.0% e.e.;
content of D-di-o-methylbenzoyltartaric acid: 0.04%;
1H-NMR (400 MHz, DMSO-d6): δ= 7.69 (s, 1H), 7.56 (s, 1H), 7.37 (d, J = 1.5 Hz, 1H), 7.28 (dd, J = 7.9, 1.5 Hz, 1H), 7.15 (d, J = 7.8 Hz, 1H), 6.72 (d, J = 29.6 Hz, 2H), 5.38 (s, 1H), 4.02 (qt, J = 7.0, 3.2 Hz, 2H), 3.83 (s, 3H), 2.16 (d, J = 25.7 Hz, 6H), 1.05 (t, J = 7.0 Hz, 3H).

### Example 2

### Example 2a

### Preparation of diastereomer salt (VII-2):

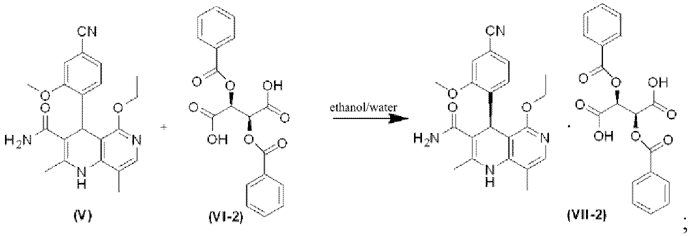

100 g (0.2642 mol) of racemic finerenone (V) was duspended in a mixture of 900 mL of ethanol and 300 mL of water, and heated to 75 °C and stirred until completely dissolved. 52.07 g (0.1453 mol) of D-dibenzoyltartaric acid was added using a solid glass funnel and stirred at 75 °C for 3.0 h for crystallization. The mixture was then cooled to 25 °C over 3 hours and stirred at this temperature for 16 hours, and the precipitated crystals were filtered out and washed twice with 200 mL of ethanol/water (3:1, v/v). The product was dried under reduced pressure at 55 °C.

Yield: 100.0 g (100.0% of th.) as off-white crystalline powder.

Analysis results:
enantiomeric purity (e.e.%): 96.7% e.e.

### Example 2b

### Preparation of crude product (Va):

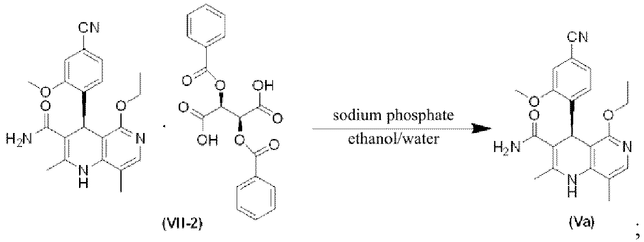

100 g of the compound prepared in Example 2a was suspended in a mixture consisting of 100 mL of ethanol and 900 mL of water. Subsequently, 367.2 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 200 mL of ethanol/water (10:90, v/v). The product was dried under reduced pressure at 55 °C. Yield: 45.0 g (87.6% of th.) as off-white crystalline powder.

Analysis results:
enantiomeric purity (e.e.%): 96.5% e.e.;
content of D-dibenzoyltartaric acid: 0.50%.

### Example 3

The crude products obtained after the dissociation of the salts with different resolving agents in Example 1b and Example 2b were tested for the residual content of the resolving agent. It was found that when D-di-o-methylbenzoyltartaric acid was used as the resolving agent, the content of the resolving agent after dissociation was less than 0.10%, which was 0.04%; and when D-dibenzoyltartaric acid was used as the resolving agent, the content of the resolving agent after dissociation was 0.50%, and two or more dissociations were required.

### Example 4

The resolution effects of Example 1a, Example 2a and (+)-O,O-di-p-anisoyl-D-tartaric acid disclosed in Patent CN112041318A were compared, and the enantiomeric excess values of different (Va) were measured and summarized in Table 2.

**Table 2**

| Resolving agent | Enantiomeric excess e.e.% (Va) |
|---|---|
| D-di-o-methylbenzoyltartaric acid | 97.2 |
| D-dibenzoyltartaric acid | 96.7 |
| (+)-O,O-di-p-anisoyl-D-tartaric acid | 68 |

As can be seen from the above table, the resolving agent of the present invention, D-di-o-methylbenzoyltartaric acid, has the best resolving effect, which is better than the resolving effects of D-dibenzoyltartaric acid and (+)-O,O-di-p-anisoyl-D-tartaric acid.

### Example 5

### Example 5a

### Preparation of diastereomer salt (VII-1):

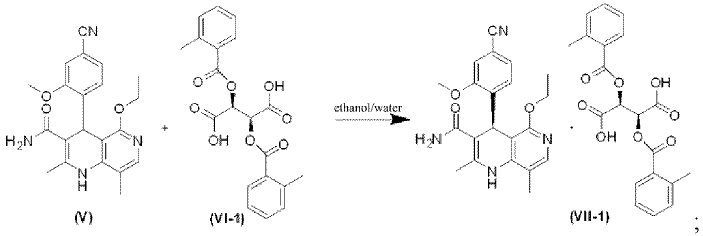

100 g (0.2642 mol) of racemic finerenone (V) was duspended in a mixture of 900 mL of ethanol and 300 mL of water, and heated to 75 °C and stirred until completely dissolved. 56.15 g (0.1453 mol) of D-di-o-methylbenzoyltartaric acid was added using a solid glass funnel and stirred at 75 °C for 1.0 h for crystallization. The mixture was then cooled to 25 °C over 3 hours and stirred at this temperature for 16 hours, and the precipitated crystals were filtered out and washed twice with 200 mL of ethanol/water (3:1, v/v). The product was dried under reduced pressure at 55 °C. Yield: 100.3 g (99.3% of th.) as off-white crystalline powder.

Analysis results:
enantiomeric purity (e.e.%): 97.0% e.e.

### Example 5b

### Preparation of crude product (Va):

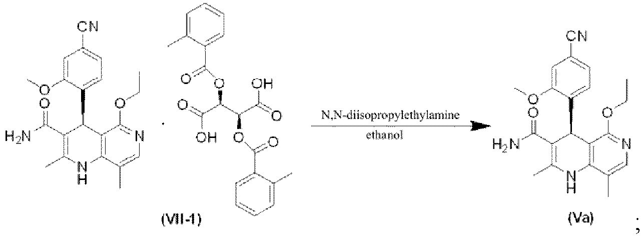

100 g of the compound (VII-1) prepared in Example 5a was suspended in 500 mL of ethanol, and 2.0 equivalents (33.80 g, 0.2615 mol) of N,N-diisopropylethylamine were added by gradually metered addition at 40 °C, and the mixture was stirred at this temperature for 1 h. After cooling to 5 °C, the mixture was stirred at this temperature for 1 h. The precipitated solid was filtered out. The product was dried under reduced pressure at 40°C. Yield: 42.1 g (85.1% of th.) as white crystalline powder.

Analysis results:
enantiomeric purity (e.e.%): 99.3% e.e.;
content of D-di-o-methylbenzoyltartaric acid: 0.08%.

### Example 5c

### Preparation of crude product (Va):

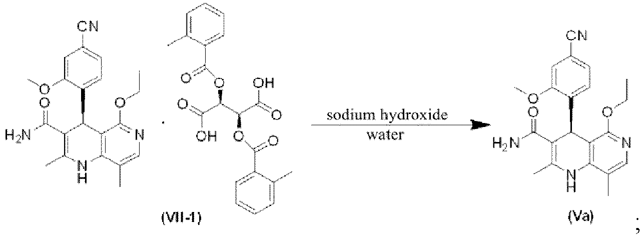

100 g of racemic finerenone (V) was used to prepare another batch of diastereomer salt (VII-1) with reference to Example 5a; and 100 g of diastereomer salt (VII-1) was suspended in 3000 mL of water. Subsequently, 88.8 g of an aqueous sodium hydroxide solution (8.8 g of sodium hydroxide dissolved in 80 g of water) was added linearly and uniformly over 10 minutes, and the pH was adjusted to pH=7.5. The mixture was heated to an internal temperature of 55°C over 1 h and stirred at this temperature for 3.0 h. The mixture was cooled to 23 °C over 1 h and stirred at this temperature for another 1 h. The precipitated crystals were filtered out and washed with 100 g of water. The wet product was then hot-slurried with 100 g of purified water at 50 °C for 1 h, cooled to 23 °C and stirred for 1 h, and the solid was filtered out, rinsed with 100 g of purified water, and dried under reduced pressure at 55 °C to obtain the final product. Yield: 45.8g (92.6% of th.) as off-white crystalline powder.

Analysis results:
enantiomeric purity (e.e.%): 98.0% e.e.;
content of D-di-o-methylbenzoyltartaric acid: Not detected.

### Example 5d

### Preparation of pure product (Va):

40 g of the crude product prepared in Example 5b was suspended in 880 mL of ethanol and then heated to reflux. Upon heating, the product was dissolved. Stirring was continued at this temperature for 1 h. The solution was filtered through a heated filter press (T = 75 °C). The solvent was then distilled until about four times the final volume (based on the material used: 40 g x 4, namely 160 mL) was obtained (about 720 mL was distilled off). The mixture was then cooled to an internal temperature of 23 °C (over about 1.5 to 2 h). The mixture was then stirred at an internal temperature of 3 °C for 2 h. The product was filtered out and rinsed once with 80 mL of absolute ethanol. The wet product was dried under reduced pressure for 48 h. Yield: 36.6 g (91.5% of th.) as colorless crystalline powder.

Analysis results:
enantiomeric purity (e.e.%): 100.0% e.e.

### Example 6

Separation of finerenone enantiomer (Vb).

### Example 6a

### Preparation of L-di-o-methylbenzoyl tartrate:

100 g (0.2642 mol) of racemic finerenone (V) was duspended in a mixture of 900 mL of ethanol and 300 mL of water, and heated to 75 °C and stirred until completely dissolved. 56.15 g (0.1453 mol) of L-di-o-methylbenzoyltartaric acid was added using a solid glass funnel and stirred at 75 °C for 3.0 h for crystallization. The mixture was then cooled to 25 °C over 3 hours and stirred at this temperature for 16 hours, and the precipitated crystals were filtered out and washed twice with 200 mL of ethanol/water (3:1, v/v). The product was dried under reduced pressure at 55 °C. Yield: 100.7 g (99.7% of th.) as off-white crystalline powder.

enantiomeric purity (e.e.%): 96.5% e.e.

### Example 6b

### Separation of finerenone (Va) from mother liquor

The combined mother liquor and washings from Example 6a were adjusted to pH = 7.5 by adding an aqueous sodium phosphate solution (100 g sodium phosphate dissolved in 1000 mL water) at room temperature. Then, distillation under reduced pressure was started to evaporate off all the ethanol, such that the residual volume was about 0.4 L. The mixture was cooled to 25 °C and stirred at 25 °C for 3 h. The product was filtered out and rinsed 4 times with 200 mL of water. The wet product was dried under reduced pressure (48 h). Yield: 46.8 g (93.6% of th.) as colorless crystalline powder.

Analysis results:
enantiomeric purity (e.e.%): 98.1% e.e.
content of L-di-o-methylbenzoyltartaric acid: 0.51%.

This crude product can be used to prepare a pure form of phenerenone (Va) in a manner similar to that described in Example 5c.

### Example 7

### Example 7a

### Preparation of diastereomer salt (VII-3):

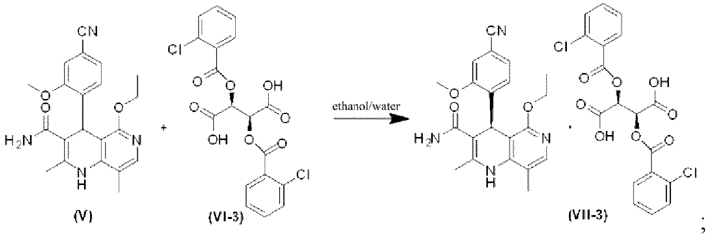

10 g (0.02642 mol) of racemic finerenone (V) was duspended in a mixture of 90 mL of ethanol and 30 mL of water, and heated to 75 °C and stirred until completely dissolved. 6.21 g (0.01454 mol) of D-di-o-chlorobenzoyltartaric acid was added using a solid glass funnel and stirred at 75 °C for 3.0 h for crystallization. The mixture was then cooled to 25 °C over 3 hours and stirred at this temperature for 16 hours, and the precipitated crystals were filtered out and washed twice with 20 mL of ethanol/water (3:1, v/v). The product was dried under reduced pressure at 55 °C.

Yield: 9.1 g (85.5% of th.) as off-white crystalline powder.

Analysis results:
enantiomeric purity (e.e.%): 90.7% e.e.

### Example 7b

### Preparation of crude product (Va):

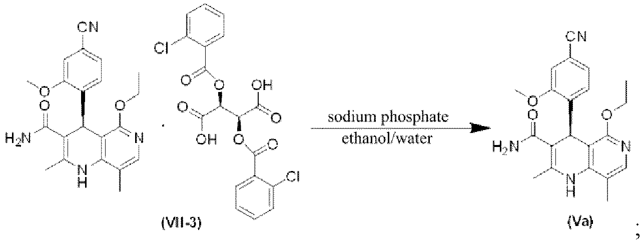

5 g of the compound prepared in Example 7a was suspended in a mixture consisting of 5 mL of ethanol and 45 mL of water. Subsequently, 10.2 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 10 mL of ethanol/water (10:90, v/v). The product was dried under reduced pressure at 55 °C. Yield: 2.1 g (89.4% of th.) as off-white crystalline powder.

Analysis results:
enantiomeric purity (e.e.%): 90.5% e.e.;
content of D-di-o-chlorobenzoyltartaric acid: 0.63%.

### Example 8

### Example 8a

### Preparation of diastereomer salt (VII-4):

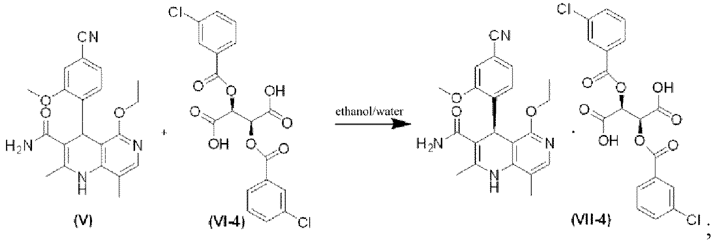

10 g (0.02642 mol) of racemic finerenone (V) was duspended in a mixture of 90 mL of ethanol and 30 mL of water, and heated to 75 °C and stirred until completely dissolved. 6.21 g (0.01454 mol) of D-di-m-chlorobenzoyltartaric acid was added using a solid glass funnel and stirred at 75 °C for 3.0 h for crystallization. The mixture was then cooled to 25 °C over 3 hours and stirred at this temperature for 16 hours, and the precipitated crystals were filtered out and washed twice with 20 mL of ethanol/water (3:1, v/v). The product was dried under reduced pressure at 55 °C.

Yield: 9.2 g (86.4% of th.) as off-white crystalline powder.

Analysis results:
enantiomeric purity (e.e.%): 88.1% e.e.

### Example 8b

### Preparation of crude product (Va):

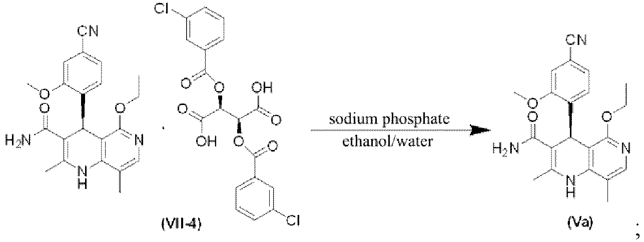

5 g of the compound prepared in Example 8a was suspended in a mixture consisting of 5 mL of ethanol and 45 mL of water. Subsequently, 10.2 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 10 mL of ethanol/water (10:90, v/v). The product was dried under reduced pressure at 55 °C. Yield: 2.0 g (85.2% of th.) as off-white crystalline powder.

Analysis results:
enantiomeric purity (e.e.%): 88.0% e.e.;
content of D-di-m-chlorobenzoyltartaric acid: 0.65%.

### Example 9

### Example 9a

### Preparation of diastereomer salt (VII-5):

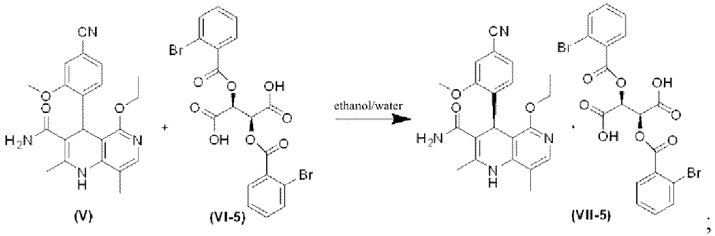

10 g (0.02642 mol) of racemic finerenone (V) was duspended in a mixture of 90 mL of ethanol and 30 mL of water, and heated to 75 °C and stirred until completely dissolved. 7.50 g (0.01454 mol) of D-di-o-bromobenzoyltartaric acid was added using a solid glass funnel and stirred at 75 °C for 3.0 h for crystallization. The mixture was then cooled to 25 °C over 3 hours and stirred at this temperature for 16 hours, and the precipitated crystals were filtered out and washed twice with 20 mL of ethanol/water (3:1, v/v). The product was dried under reduced pressure at 55 °C.

Yield: 10.3 g (87.1% of th.) as off-white crystalline powder.

Analysis results:
enantiomeric purity (e.e.%): 93.1% e.e.

### Example 9b

### Preparation of crude product (Va):

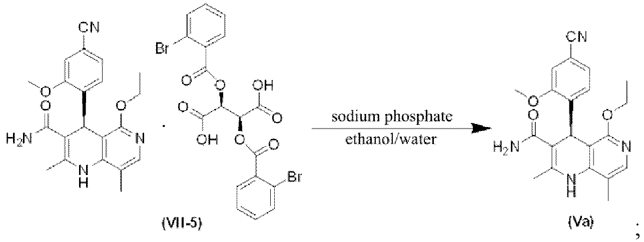

5 g of the compound prepared in Example 9a was suspended in a mixture consisting of 5 mL of ethanol and 45 mL of water. Subsequently, 10.1 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 10 mL of ethanol/water (10:90, v/v). The product was dried under reduced pressure at 55 °C. Yield: 1.9 g (89.8% of th.) as off-white crystalline powder.

Analysis results:
enantiomeric purity (e.e.%): 93.3% e.e.;
content of D-di-o-bromobenzoyltartaric acid: 0.35%.

### Example 10

### Example 10a

### Preparation of diastereomer salt (VII-6):

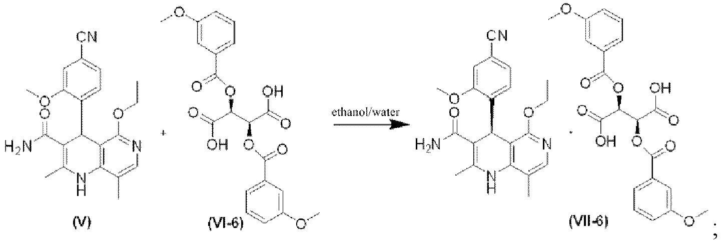

10 g (0.02642 mol) of racemic finerenone (V) was duspended in a mixture of 90 mL of ethanol and 30 mL of water, and heated to 75 °C and stirred until completely dissolved. 6.08 g (0.01454 mol) of D-di-m-methoxybenzoyltartaric acid was added using a solid glass funnel and stirred at 75 °C for 3.0 h for crystallization. The mixture was then cooled to 25 °C over 3 hours and stirred at this temperature for 16 hours, and the precipitated crystals were filtered out and washed twice with 20 mL of ethanol/water (3:1, v/v). The product was dried under reduced pressure at 55 °C.

Yield: 9.9 g (94.0% of th.) as off-white crystalline powder.

Analysis results:
enantiomeric purity (e.e.%): 95.6% e.e.

### Example 10b

### Preparation of crude product (Va):

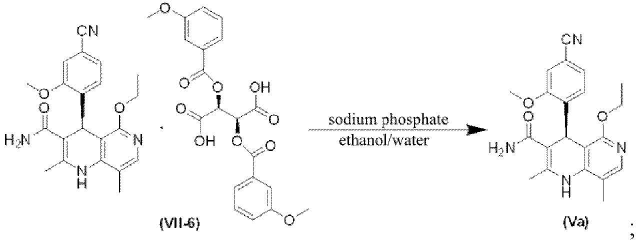

5 g of the compound prepared in Example 10a was suspended in a mixture consisting of 5 mL of ethanol and 45 mL of water. Subsequently, 10.3 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 10 mL of ethanol/water (10:90, v/v). The product was dried under reduced pressure at 55 °C. Yield: 2.2 g (92.6% of th.) as off-white crystalline powder.

Analysis results:
enantiomeric purity (e.e.%): 95.5% e.e.;
content of D-di-m-methoxybenzoyltartaric acid: 0.13%.

### Example 11

### Example 11a

### Preparation of diastereomer salt (VII-7):

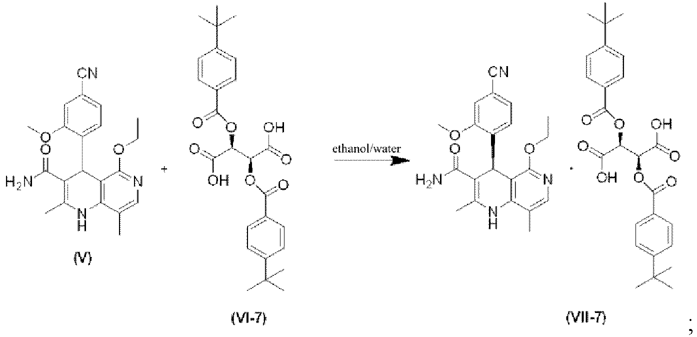

10 g (0.02642 mol) of racemic finerenone (V) was duspended in a mixture of 90 mL of ethanol and 30 mL of water, and heated to 75 °C and stirred until completely dissolved. 6.84 g (0.01454 mol) of D-di-p-tert-butylbenzoyltartaric acid was added using a solid glass funnel and stirred at 75 °C for 3.0 h for crystallization. The mixture was then cooled to 25 °C over 3 hours and stirred at this temperature for 16 hours, and the precipitated crystals were filtered out and washed twice with 20 mL of ethanol/water (3:1, v/v). The product was dried under reduced pressure at 55 °C.

Yield: 10.5 g (93.6% of th.) as off-white crystalline powder.

Analysis results:
enantiomeric purity (e.e.%): 92.7% e.e.

### Example 11b

### Preparation of crude product (Va):

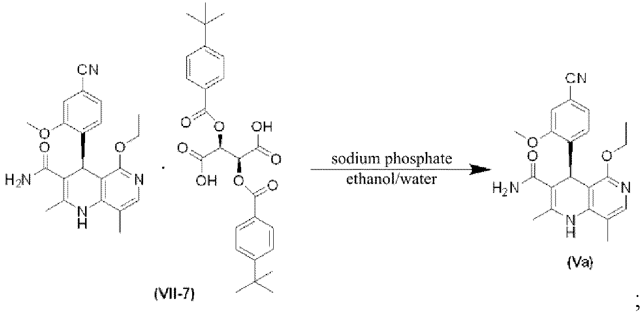

5 g of the compound prepared in Example 11a was suspended in a mixture consisting of 5 mL of ethanol and 45 mL of water. Subsequently, 10.2 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 10 mL of ethanol/water (10:90, v/v). The product was dried under reduced pressure at 55 °C. Yield: 1.9 g (85.2% of th.) as off-white crystalline powder.

Analysis results:
enantiomeric purity (e.e.%): 92.7% e.e.;
content of D-di-p-tert-butylbenzoyltartaric acid: 0.45%.

### Example 12

### Example 12a

### Preparation of diastereomer salt (VII-8):

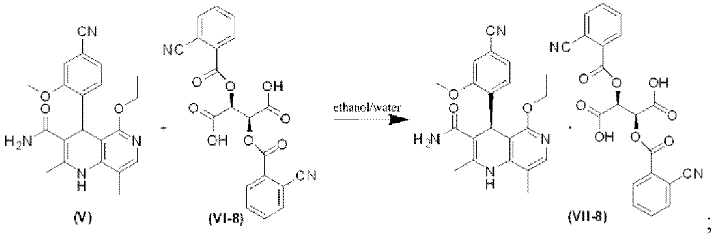

10 g (0.02642 mol) of racemic finerenone (V) was duspended in a mixture of 90 mL of ethanol and 30 mL of water, and heated to 75 °C and stirred until completely dissolved. 5.94 g (0.01454 mol) of D-di-o-cyanobenzoyltartaric acid was added using a solid glass funnel and stirred at 75 °C

for 3.0 h for crystallization. The mixture was then cooled to 25 °C over 3 hours and stirred at this temperature for 16 hours, and the precipitated crystals were filtered out and washed twice with 20 mL of ethanol/water (3:1, v/v). The product was dried under reduced pressure at 55 °C.

Yield: 9.5 g (91.4% of th.) as off-white crystalline powder.

Analysis results:
enantiomeric purity (e.e.%): 92.9% e.e.

### Example 12b

### Preparation of crude product (Va):

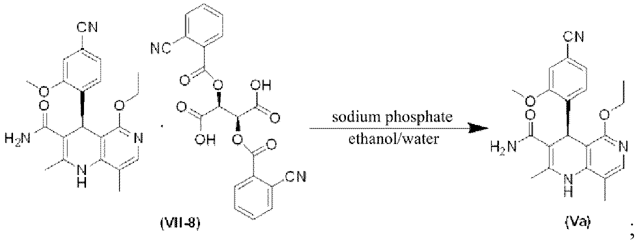

5 g of the compound prepared in Example 12a was suspended in a mixture consisting of 5 mL of ethanol and 45 mL of water. Subsequently, 10.4 g of an aqueous sodium phosphate solution (100 g of sodium phosphate dissolved in 1000 mL of water) was added linearly and uniformly over 1 h, and the pH was adjusted to pH = 7.5. The mixture was heated to an internal temperature of 55°C over 1 h and stirred at this temperature for 3.0 h. The mixture was cooled to 20 °C over 1 h and stirred at this temperature for another 1 h. The precipitated crystals were filtered out and washed twice with 10 mL of ethanol/water (10:90, v/v). The product was dried under reduced pressure at 55 °C. Yield: 2.2 g (91.5% of th.) as off-white crystalline powder.

Analysis results:
enantiomeric purity (e.e.%): 92.6% e.e.;
content of D-di-o-cyanobenzoyltartaric acid: 0.15%.

## Claims

1. A method for resolving a racemate represented by formula (V), wherein the method uses a chiral substituted tartaric ester represented by general formula (VIa) or (VIb) as a resolving agent to resolve a racemate represented by formula (V) to obtain a compound represented by formula (Va) and/or (Vb), 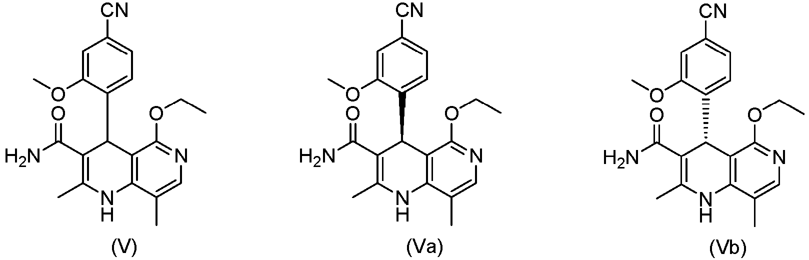 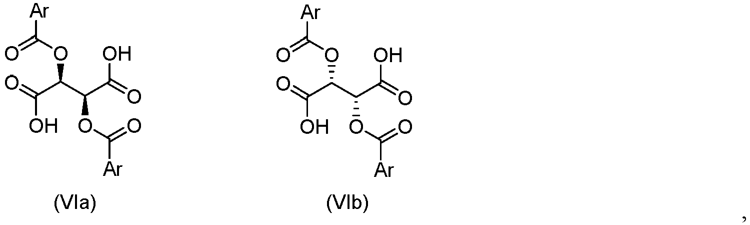 wherein Ar is selected from: 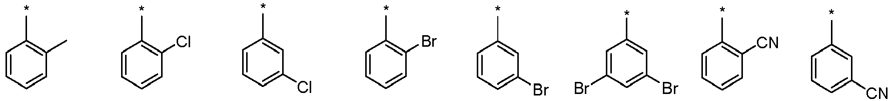 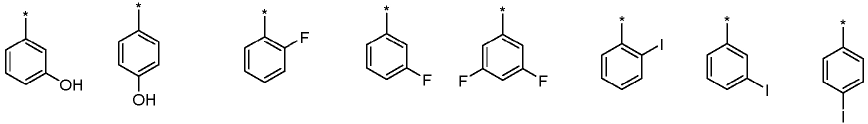 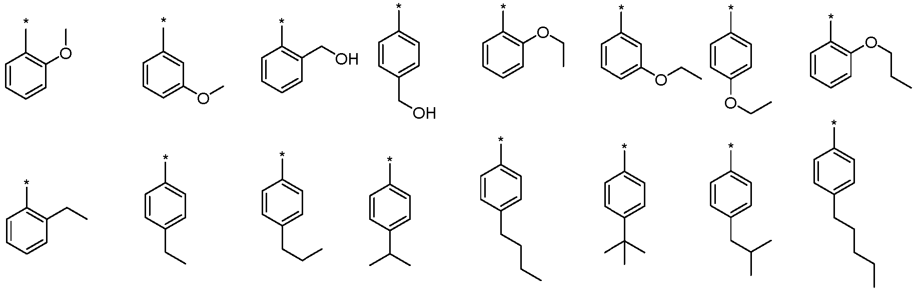 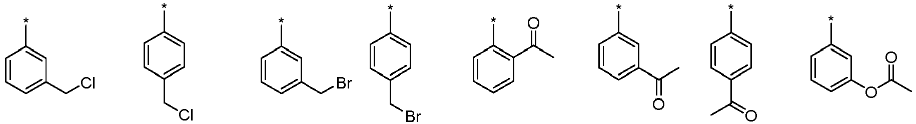 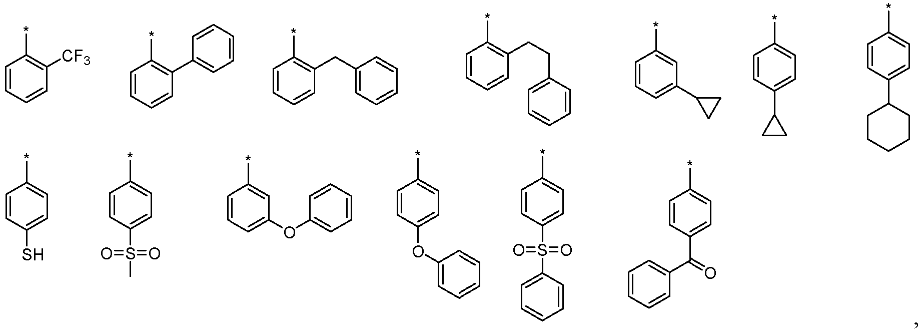 wherein * represents an attachment point.

2. A method for preparing (4S)-4-(4-cyano-2-methoxyphenyl)-5-ethoxy-2,8-dimethyl-1,4-dihydro-1,6-naphthyridine-3-carboxamide represented by formula (Va), wherein the method uses a chiral substituted tartaric ester represented by formula (VIa) as a resolving agent to resolve a racemate represented by formula (V), 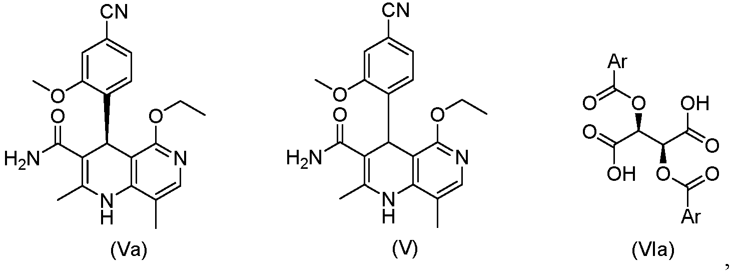 wherein Ar is selected from: 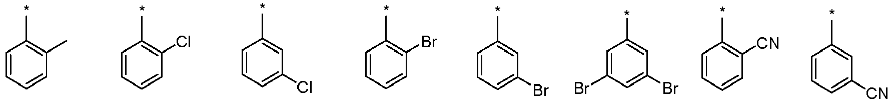 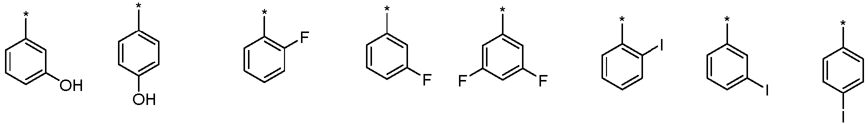 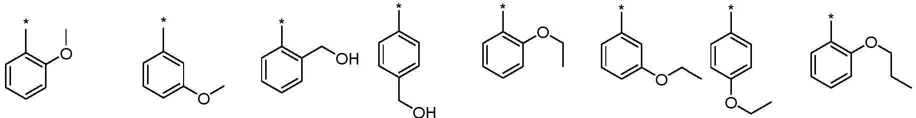 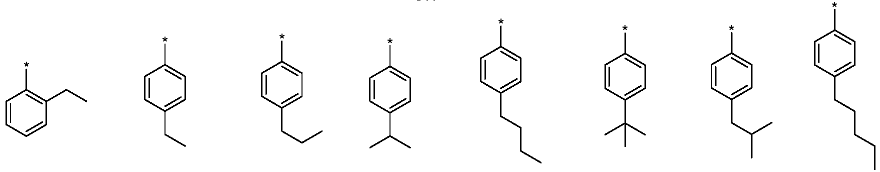 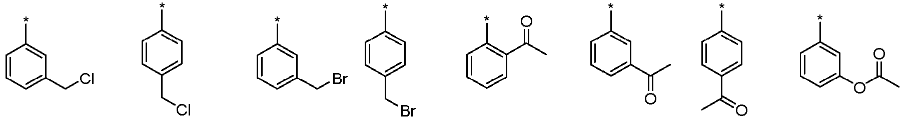 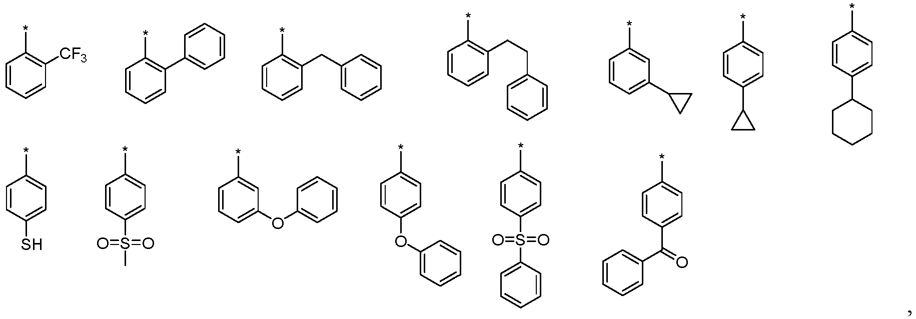 wherein * represents an attachment point.

3. The method according to claim 1 or 2, wherein the resolution of the racemate is carried out in an ethanol/water binary solvent system.

4. The method according to claim 3, wherein the resolution of the racemate is carried out at a temperature ranging from 60 to 80 °C.

5. The method according to claim 4, wherein the resolving agent is selected from:

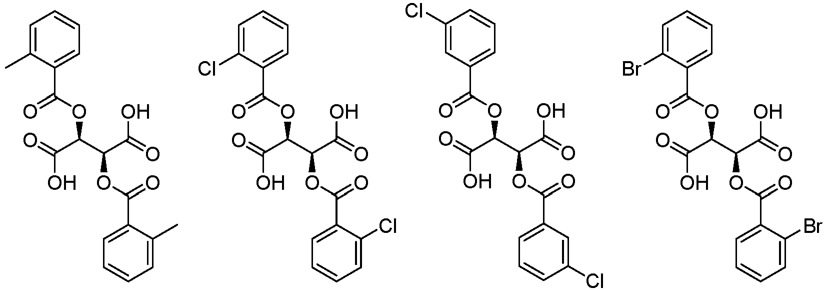

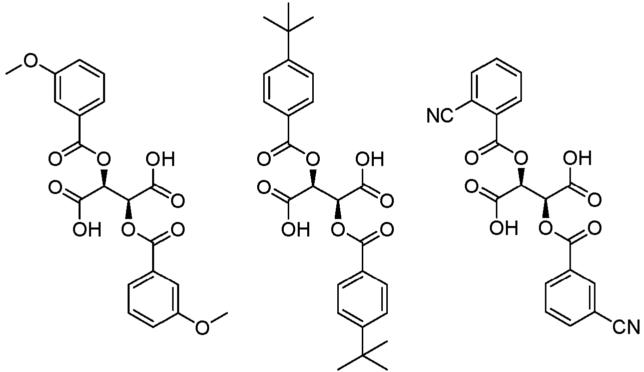

6. The method according to claim 5, wherein the resolving agent is selected from:

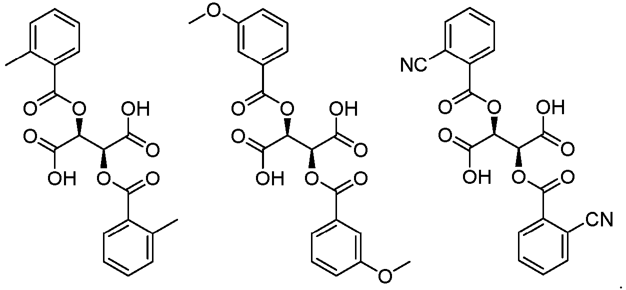

7. The method according to claim 1 or 2, wherein the precipitated diastereomer salts (VIIa), (VIIb), (VIIc) and/or (VIId) are separated.

8. The method according to claim 7, wherein the diastereomer salt is treated with a base and the solvent is removed.

9. The method according to claim 8, wherein the base is an inorganic base selected from ammonia, sodium hydroxide, lithium hydroxide, potassium hydroxide, ammonium carbonate, sodium carbonate, potassium carbonate, lithium carbonate, ammonium hydrogen carbonate, sodium hydrogen carbonate, potassium hydrogen carbonate, ammonium phosphate, sodium phosphate or potassium phosphate.

10. The method according to claim 8, wherein the base is an organic base selected from methylamine, dimethylamine, trimethylamine, ethylamine, diethylamine, triethylamine, propylamine, isopropylamine, 1,3-propylenediamine, 1,2-propylenediamine, tripropylamine, cyclohexylamine, dicyclohexylamine, N,N-diisopropylethylamine, aniline, diphenylamine, triphenylamine, monoethanolamine, diethanolamine, triethanolamine, N-methylmorpholine, pyridine, sodium tert-butoxide, potassium tert-butoxide, sodium ethoxide or sodium methoxide.

11. The method according to claim 8, wherein the racemate represented by formula (V) is reacted with D-di-o-methylbenzoyltartaric acid represented by formula (VI-1), 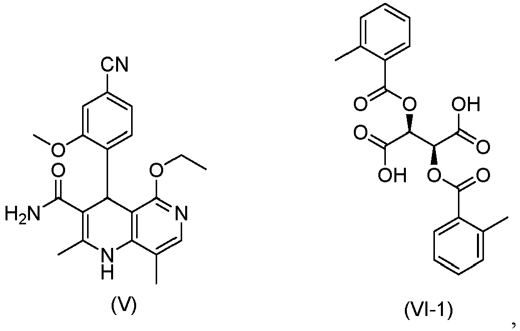 in an ethanol/water binary solvent system to obtain a diastereomer salt represented by formula (VII-1), 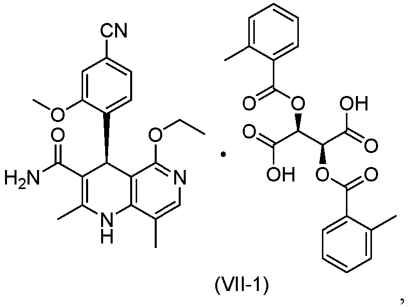 and subsequently, finerenone (Va) is dissociated using an aqueous sodium phosphate or sodium hydroxide solution in an ethanol/water binary solvent system,

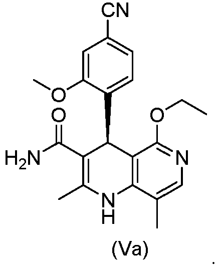

12. Diastereomer salts represented by the following formulae, 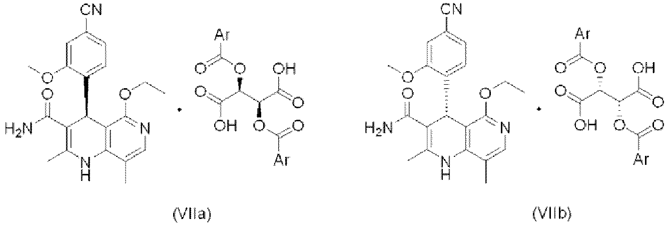 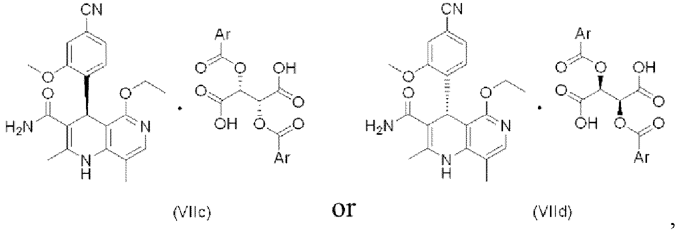 wherein Ar is selected from: 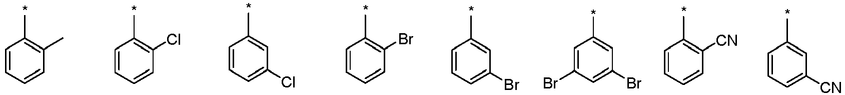 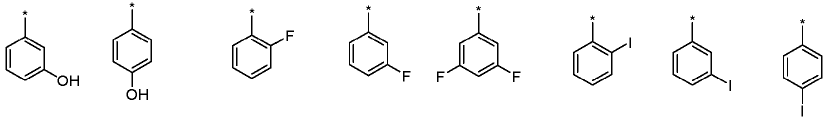 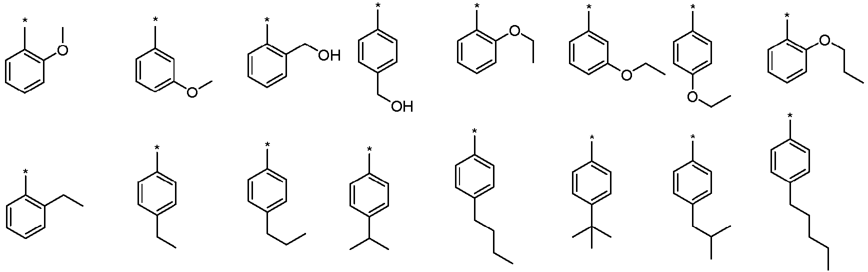 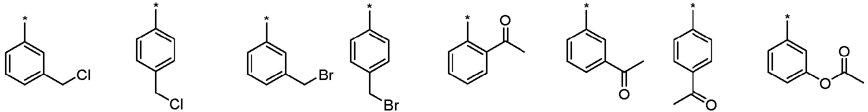 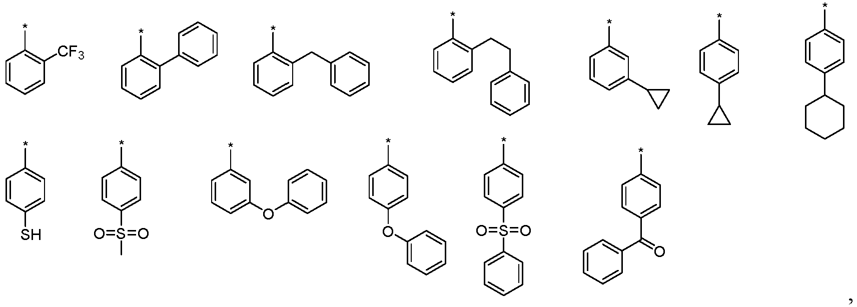 wherein * represents an attachment point.

13. A diastereomer salt represented by formula (VII-1),

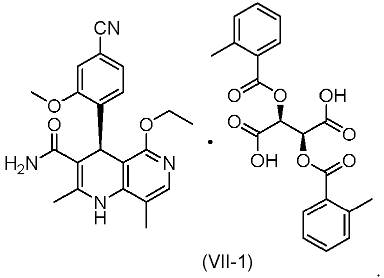

14. The diastereomer salt according to claim 13, wherein the diastereomer salt represented by formula (VII-1) is in a crystalline form having a XRPD spectrum with diffraction peaks at 2θ of 8.2° ± 0.2°, 11.1° ± 0.2°, 14.6° ± 0.2°, 14.9° ± 0.2°, 16.6° ± 0.2°, 16.9° ± 0.2°, 18.8° ± 0.2°, 19.2° ± 0.2°, 21.9° ± 0.2° and 25.7° ± 0.2°.
